# FASCICULE DE BREVET EUROPEEN

(11) **EP 1 857 143 B1**
(45) Date de publication et mention de la délivrance du brevet: **26.03.2008**
(21) Numéro de dépôt: 07290606.8
(22) Date de dépôt: 14.05.2007
(51) Int. Cl.: A61N 1/365, A61N 1/37, A61N 1/08

(54) **Dispositif médical implantable actif de stimulation cardiaque resynchronisation cardioversion et/ou défibrillation comportant des moyens de détection de fracture de sonde**
Aktives medizinisches Implantat zur Herzstimulation, Resynchronisation, Kardioversion und/oder Defibrillation, das Mittel zum Erfassen eines Sondenbruchs umfasst
Active implantable medical device for cardiac stimulation, resynchronisation, cardioversion and/or defibrillation, comprising means for detecting catheter fractures

(30) Priorité: 18.05.2006 FR 0604445
(43) Date de publication de la demande: 21.11.2007
(73) Titulaire: ELA MEDICAL, 92541 Montrouge (FR)
(72) Inventeur: Vincent, Elodie, 92160 Antony (FR); Amblard, Amel, 92290 Chatenay Malabry (FR)
(74) Mandataire: Dupuis-Latour, Dominique

(56) Documents cités:
- EP-A- 1 433 496
- EP-A- 1 438 985
- EP-A- 1 537 894
- EP-A2- 0 655 260
- US-A- 5 755 742
- US-A- 5 776 168
- US-A- 5 814 088
- US-B2- 6 892 092

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation, de cardioversion et/ou de défibrillation en cas de trouble du rythme détecté par le dispositif.

L'analyse du rythme cardiaque est effectuée à partir de signaux d'électrogramme (EGM), recueillis par des électrodes portées par des sondes endocavitaires implantées dans le myocarde pour mesurer le potentiel de dépolarisation auriculaire et/ou ventriculaire. Ces signaux sont analysés par l'implant, qui délivrera éventuellement au patient une thérapie appropriée sous forme d'impulsions de faible énergie (stimulation antibradycardique ou stimulation de resynchronisation des ventricules) ou de chocs de cardioversion ou de défibrillation.

Les sondes implantées qui portent les électrodes ont généralement un très faible diamètre et une grande flexibilité, pour supporter les contraintes mécaniques permanentes auxquelles elles sont soumises au rythme des battements du coeur. Il peut arriver cependant que, malgré leurs caractéristiques mécaniques, ces sondes présentent à la longue une dégradation de l'isolant externe susceptible de perturber la détection du signal. On estime ainsi que près de 10% des porteurs de défibrillateurs implantables présentent des fractures d'isolant ou de conducteur susceptibles d'altérer la détection du rythme cardiaque intrinsèque.

On englobera sous le terme de "fracture" ces divers types de dégradations, incluant aussi bien les fractures proprement dites que les amorces de fracture apparaissant aux premiers stades du phénomène. En effet, la fracture de sonde peut apparaître de façon très progressive, tout d'abord par une fissuration de l'isolant : les potentiels électrochimiques produits à l'endroit de la rupture sont alors susceptibles de parasiter le signal de détection du rythme cardiaque, perturbation qui pourrait être analysée à tort comme une dépolarisation réelle du ventricule.

Ce phénomène est d'autant plus pernicieux qu'il apparaît souvent de façon très progressive, en ne perturbant au début que quelques cycles ; de plus, si le phénomène est synchrone à la contraction, il peut être occulté pendant une durée relativement longue, et même passer inaperçu lors des examens de suivi du patient effectués par un électrophysiologiste analysant directement, en temps réel, les signaux produits par le dispositif implanté au moyen d'un programmateur externe. D'autre part, outre son caractère intermittent, la fracture peut affecter le conducteur de masse, de sorte qu'avec une sonde bipolaire elle ne pourra pas être immédiatement détectée. Ce n'est que quand la rupture est totale que le dispositif la détecte, du fait de l'absence de tout signal recueilli en entrée.

Dans l'intervalle, les risques de surdétection ventriculaire peuvent leurrer le dispositif, avec le risque de générer des thérapies inappropriées, par exemple en inhibant à tort les stimulations antibradycardiques ou les thérapies de resynchronisation ou, inversement, en délivrant à tort des chocs à haute énergie inappropriés sur diagnostic erroné d'une tachycardie ou d'une fibrillation, chocs douloureux pour le patient et qui peuvent être délétères.

Le problème de l'invention consiste à détecter les risques de fracture de sonde lorsque ces fractures ne se manifestent encore que de manière intermittente.

Plus précisément, le problème de l'invention consiste à discriminer les perturbations électriques générées par une fracture de sonde parmi le dépolarisations cardiaques effectivement recueillies par la sonde implantée, ceci afin d'éviter le déclenchement de traitements inappropriés et/ou pour produire une alarme bien avant que la fracture se manifeste de façon totale et continue.

Le point de départ de l'invention est la constatation de ce que la dépolarisation, qui est un phénomène électrique sensible au bruit, est normalement suivie d'une contraction cardiaque, qui est un phénomène mécanique qui n'est pas affecté par le bruit. De la sorte, en procédant à une double détection - de la dépolarisation et de la contraction - par des moyens distincts, en présence de perturbations suspectes telles que celles produites par une fracture de sonde on peut opérer un lever de doute pour confirmer que le signal détecté a été effectivement suivi par une activité mécanique du coeur et constitue donc bien un signal de dépolarisation et non une perturbation liée à une fracture de sonde.

La détection de l'activité mécanique du coeur peut en particulier être opérée par mesure de l'accélération endocardiaque, réalisée par un accéléromètre directement en contact avec le muscle cardiaque (généralement à l'apex ventriculaire droit).

On sait en effet que l'accélération endocardiaque reflète très précisément et en temps réel les phénomènes concourant au fonctionnement mécanique du coeur.

Plus précisément, le EP-A-0 515 319 (Sorin Biomedica Cardio SpA) enseigne la manière de recueillir un signal d'accélération endocardiaque au moyen d'une sonde endocavitaire pourvue d'une électrode distale de stimulation implantée au fond du ventricule et intégrant un micro-accéléromètre permettant de mesurer l'accélération endocardiaque.

Le signal d'accélération endocardiaque ainsi recueilli au cours d'un cycle cardiaque forme notamment deux pics correspondant aux deux bruits majeurs qu'il est possible de reconnaître dans chaque cycle d'un coeur sain :
- le premier pic d'accélération endocardiaque ("PEA I") correspond à la fermeture des valvules mitrale et tricuspide, au début de la phase de contraction ventriculaire isovolumique (systole). Les variations de ce premier pic sont étroitement liés aux variations de la pression dans le ventricule (l'amplitude du pic PEA I étant, plus précisément, corrélée au maximum positif de la variation de pression dP/dt dans le ventricule gauche) et peuvent donc constituer un paramètre représentatif de la contractilité du myocarde.
- le second pic d'accélération endocardiaque ("PEA II"), quant à lui, correspond à la fermeture des valvules aortique et pulmonaire, au début de la diastole. Il est produit par la décélération brusque de la masse sanguine en mouvement dans l'aorte.

Le EP-A-0 655 260 (Sorin Biomedica Cardio SpA) décrit une manière de traiter le signal d'accélération endocavitaire délivré par le capteur situé en bout de sonde pour en dériver deux valeurs respectives liées à ces pics d'accélération endocardiaque.

Dans ce document, il est proposé d'utiliser les valeurs d'amplitude des pics PEA I et PEA II pour détecter des troubles cardiaques et déclencher ou non une thérapie de défibrillation.

Dans le cas de la présente invention, il s'agit de détecter la présence ou l'absence d'une contraction cardiaque, en partant du principe qu'à chaque cycle cardiaque réel correspond une seule contraction cardiaque. L'accélération endocardiaque est analysée, avantageusement en détectant la présence ou non d'un pic PEA I, pour confirmer la présence d'une activité mécanique du coeur sur détection d'une dépolarisation : une telle détection qui ne serait pas suivie par une activité mécanique du coeur peut avoir été générée par une perturbation produite par une fracture de sonde, elle est donc suspecte et doit être diagnostiquée comme telle.

Le dispositif de l'invention est du type décrit par le EP-A-0 655 260 précité, correspondant au préambule de la revendication 1, et il comprend en outre les moyens énoncés par la partie caractéristique de cette revendication 1. Les sous-revendications visent des formes de mise en oeuvre avantageuses.

On va maintenant décrire un exemple de mise en oeuvre du dispositif de l'invention, en référence aux dessins annexés.
La figure 1 est un chronogramme montrant, au cours de trois cycles cardiaques successifs, les variations de l'accélération endocavitaire ainsi que de l'électrogramme et de l'électrocardiogramme de surface.
La figure 2 est un autre chronogramme montrant, au cours de six cycles cardiaques successifs, les différents signaux recueillis représentatifs de dépolarisations successives et les signaux indiquant la présence d'un pic d'accélération endocardiaque.
La figure 3 est un organigramme illustrant la succession des différentes étapes d'analyse pour la mise en oeuvre de l'invention.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque ou défibrillateur/cardioverteur, comprenant des moyens d'acquisition d'un signal fourni par des sondes endocavitaires et/ou un ou plusieurs capteurs implantés.

L'invention peut notamment être appliquée aux dispositifs implantables commercialisés par ELA Médical, Montrouge, France tels que les appareils *Symphony* et *ELA Rhapsody.*

Il s'agit de dispositifs à microprocesseur programmables comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

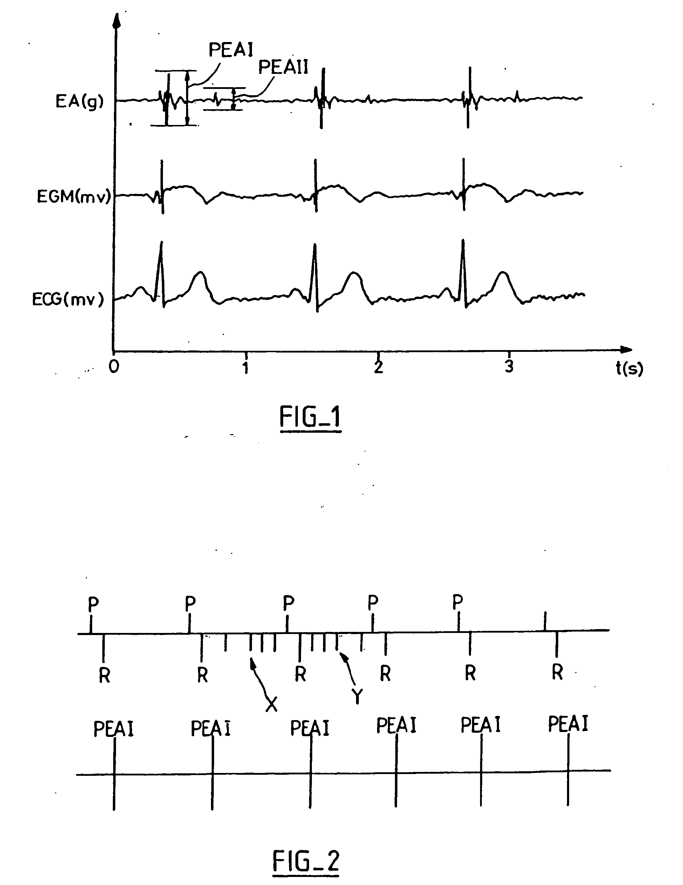

Sur la figure 1, on a représenté (tracé du haut), les variations de l'accélération endocardiaque (EA), mesurée par un capteur tel que celui décrit dans le EP-A-0 515 319 précité, intégré à une tête de sonde endocavitaire. On a également illustré sur cette figure les tracés de l'électrogramme (EGM), c'est-à-dire du signal électrique capté par une électrode endocardiaque de recueil, ainsi que d'un électrocardiogramme de surface (ECG) correspondant, au cours de trois cycles cardiaques consécutifs. Comme on l'a expliqué plus haut le tracé de l'accélération présente deux complexes successifs ou pics d'accélération endocardiaque (PEA), dont les paramètres (amplitude, durée et position temporelle, c'est-à-dire instant de survenue) peuvent être déterminées par un traitement approprié du signal délivré par le capteur d'accélération, comme décrit dans le EP-A-0 655 260 précité.

La présente invention propose d'utiliser les paramètres liés à l'accélération endocardiaque ainsi recueillis, notamment la survenue du pic PEA I (indiquée par la position temporelle de ce pic), pour confirmer ou infirmer la présence d'une activité mécanique du coeur.

La première ligne de la figure 2 illustre la succession des évènements auriculaires P et ventriculaires R au cours de six cycles cardiaques successifs, pour un patient présentant un rythme sinusal normal.

Le recueil de ces signaux peut être perturbé par des potentiels électrochimiques apparaissant à l'endroit d'une fracture ou amorce de fracture de la sonde, potentiels qui peuvent se traduire par des perturbations, telles que celles illustrées en X et Y, susceptibles d'être interprétés à tort par le dispositif comme des évènements ventriculaires, conduisant à une suspicion erronée d'augmentation brutale du rythme ventriculaire, similaire à ce qui pourrait apparaître en cas de fibrillation ventriculaire.

En revanche, la séquence des pics d'accélération (seconde ligne de la figure 2) n'est pas perturbée par une possible fracture ou amorce de fracture, puisqu'il s'agit de la détection d'une activité purement mécanique, comme on l'a expliqué plus haut. Le caractère régulier des contractions permet d'écarter le diagnostic de fibrillation ventriculaire et de suspecter la présence d'une fracture sur la sonde.

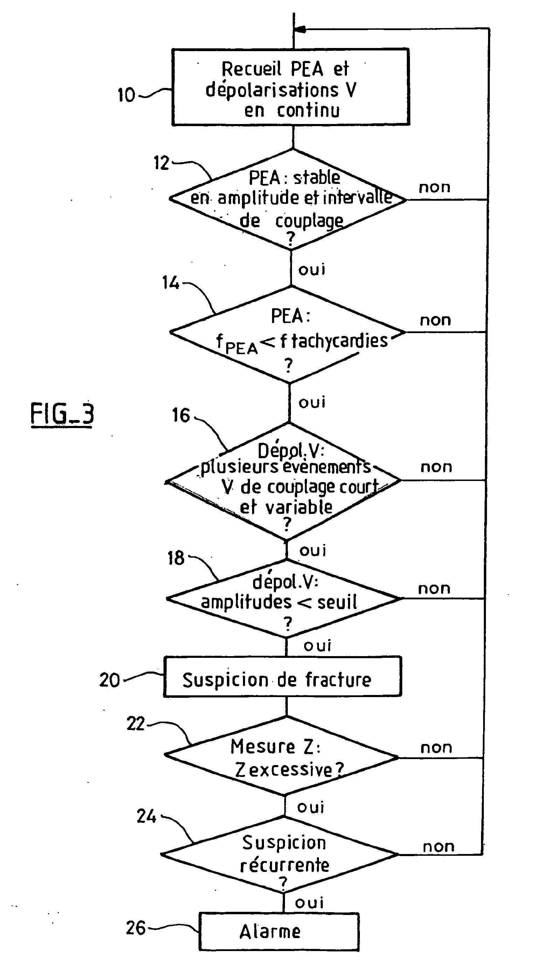

On va maintenant décrire plus en détail, en référence à l'organigramme de la figure 3, la manière dont est opérée la corrélation entre les signaux représentatifs des dépolarisations (première ligne de la figure 2) et ceux représentatifs des pics d'accélération (seconde ligne de la figure 2). Pour ce faire, la sonde endocavitaire portant le capteur d'accélération endocardiaque est différente de la sonde recueillant les signaux EGM.

La première étape, référencée 10, consiste à recueillir de façon continue les signaux d'accélération endocardiaque et les dépolarisations ventriculaires, l'analyse étant opérée sur chaque cycle cardiaque.

Le dispositif détermine à partir de ces mesures une première série de signaux représentatifs des dépolarisations ventriculaires, et une deuxième série de signaux représentatifs des pics d'accélération (avantageusement le pic PEA I).

Le dispositif peut notamment utiliser les signaux d'accélération endocardiaque au niveau du ventricule droit. Mais l'invention peut être également mise en oeuvre en utilisant des signaux représentatifs de l'accélération endocardiaque relevée au niveau :
- d'une oreillette,
- ou du ventricule gauche,
- ou d'un vaisseau sanguin périphérique du coeur, c'est-à-dire d'un vaisseau situé sur le coeur ou à proximité immédiate du coeur (en contact avec la paroi du coeur).

La première phase de l'analyse (étape 12) consiste à déterminer si les signaux de pics PEA sont stables en amplitude et/ou en intervalles de couplage (l'intervalle de couplage étant la période temporelle séparant deux pics relatifs à des cycles consécutifs). La condition de stabilité en amplitude signifie par exemple que l'amplitude du pic PEA I ne varie pas de plus de x % par rapport à la moyenne des y cycles précédents. La condition de stabilité du couplage signifie que l'intervalle de couplage ne varie pas de plus ou moins z millisecondes, par exemple plus ou moins 30 millisecondes d'un cycle au suivant.

En présence d'un rythme PEA stable révélateur de contractions régulières, le dispositif détermine (étape 14) si la fréquence de ces contractions (fréquence des pics PEA) est inférieure à une fréquence limite, inférieure à la zone de détection des tachycardies.

Dans la négative, il s'agit vraisemblablement d'une tachycardie avérée, pour laquelle un thérapie doit être envisagée sans qu'il y ait lieu de poursuivre l'analyse.

Dans l'affirmative, donc en présence d'un rythme des contractions suffisamment lent, le dispositif examine (étape 16) s'il est en présence d'une série d'évènements ventriculaires de couplage court et variable (le critère de "couplage court" signifiant que les intervalles de couplage entre événements ventriculaires successifs sont inférieurs à un seuil donné, et le critère de "couplage variable" signifiant que les différences entre les intervalles de couplage dépassent un seuil donné sur un nombre prédéterminé de cycles successifs).

Si l'analyse des dépolarisations ventriculaires révèle à l'étape 16 un rythme rapide et instable, le dispositif détermine si les amplitudes de ces dépolarisations sont inférieures ou non à un seuil donné (étape 18). Dans l'affirmative, il est probable que ces signaux ne représentent pas de véritables dépolarisations, et le diagnostic de fracture n'est pas poursuivi. Il est également possible, à cette étape, d'effectuer le test sur une pluralité d'évènements ventriculaires, le test consistant à déterminer le nombre d'évènements ventriculaires présentant une amplitude inférieure au seuil prédéterminé, et à n'abandonner la poursuite du diagnostic que si le nombre de ces évènements est supérieur à un nombre donné, ceci afin d'éviter que le diagnostic ne soit interrompu par un nombre réduit d'évènements atypiques.

Si les conditions établies aux étapes 12 à 18 sont vérifiées, alors le dispositif détermine qu'il y a suspicion de fracture (étape 20), par exemple en positionnant un indicateur spécifique.

Cette suspicion de fracture peut être en particulier utilisée pour déclencher une mesure d'impédance de la sonde (étape 22) par un procédé connu, par exemple du type exposé dans le EP-A-1 216 723 (ELA Medical) qui décrit un circuit permettant d'évaluer l'impédance complexe d'une sonde par application d'impulsions de stimulation spécifiques et analyse des variations résultantes du signal détecté.

Si cette mesure d'impédance révèle effectivement un défaut (étape 22), et que, en tout état de cause, le diagnostic de suspicion de fracture est un diagnostic récurrent (étape 24), alors le dispositif considère qu'il y a une fracture avérée et produit un signal d'alarme (étape 26).

L'étape 24 consistant à vérifier si la suspicion est récurrente ou non permet d'éliminer le cas de certains signaux parasites survenant de manière ponctuelle en produisant des artefacts de détection qui ne sont pas liés à une fracture de sonde. En effet, une fracture apparaît généralement de manière progressive et, tout au moins au début, de manière intermittente. C'est la répétition des perturbations produites par les courants de rupture qui va permettre de confirmer qu'il s'agit bien d'une fracture de sonde, et non d'artefacts de bruit extrinsèque, tels que les interférences électromagnétiques provenant d'équipements électroniques de surveillance, d'appareils électriques environnants, d'instruments électrochirurgicaux de systèmes de communication, etc.

L'alarme produite à l'étape 26 peut comprendre notamment :
- l'enregistrement d'un marqueur dans une mémoire du dispositif, permettant de signaler à l'électrophysiologiste lors d'une visite de routine ultérieure qu'un phénomène de fracture a été diagnostiqué par l'appareil, et/ou
- la production par un "buzzer" d'un signal sonore à destination du patient, de manière à alerter celui-ci sans délai, et/ou
- l'émission d'un signal par des moyens de transmission RF.

## Revendications

1. Un dispositif médical implantable actif du type prothèse cardiaque de stimulation, resynchronisation, cardioversion et/ou défibrillation, comportant :
- des moyens de détection du rythme, comprenant une sonde implantable pourvue d'au moins une électrode endocavitaire apte à recueillir des potentiels électriques représentatifs de dépolarisations du myocarde, et un circuit de détection apte à analyser les potentiels recueillis et délivrer une séquence de signaux représentatifs des dépolarisations ventriculaires et auriculaire (P, R) successives, et
- des moyens de détection des contractions du myocarde comprenant un capteur d'accélération endocardiaque, et des moyens pour déterminer au moins un pic de l'accélération endocardiaque au cours d'un cycle cardiaque donné et délivrer une séquence de signaux représentatifs des pics d'accélération (PEA I) successifs,
dispositif **caractérisé en ce qu**'il comporte en outre des moyens de détection de fracture de ladite sonde, comprenant des moyens pour :
- recevoir en entrée et corréler entre eux lesdits signaux représentatifs des dépolarisations et lesdits signaux représentatifs des pics d'accélération, et
- en cas de défaut de corrélation, délivrer un indicateur de suspicion de fracture (20) de ladite sonde.

2. Le dispositif de la revendication 1, comprenant deux sondes endocavitaires distinctes, avec une première sonde portant ladite électrode endocavitaire et une deuxième sonde portant ledit capteur d'accélération endocardiaque, lesdits moyens de détection de fracture étant des moyens de détection d'une fracture de ladite première sonde.

3. Le dispositif de la revendication 1, comprenant en outre des moyens de mesure d'impédance de sonde (22), activables en réponse à la délivrance d'un indicateur de suspicion de fracture.

4. Le dispositif de la revendication 1, comprenant en outre des moyens pour produire un signal d'alarme (26) en réponse à la délivrance récurrente d'indicateurs de suspicion de fracture sur une durée prédéterminée.

5. Le dispositif de la revendication 4, dans lequel les moyens pour produire un signal d'alarme (26) comprennent des moyens d'enregistrement de marqueurs dans une mémoire du dispositif lisible par un programmateur externe.

6. Le dispositif de la revendication 4, dans lequel les moyens pour produire un signal d'alarme (26) comprennent des moyens de transmission RF.

7. Le dispositif de la revendication 4, dans lequel les moyens pour produire un signal d'alarme (26) comprennent des moyens de production d'un signal sonore.

8. Le dispositif de la revendication 1, dans lequel la délivrance d'un indicateur de suspicion de fracture (20) est conditionnée à la détection (12) d'une séquence de pics d'accélération stable en amplitude et/ou en intervalles de couplage.

9. Le dispositif de la revendication 1, dans lequel la délivrance d'un indicateur de suspicion de fracture (20) est conditionnée à la détection (14) d'une séquence de pics d'accélération de fréquence inférieure à une fréquence limite représentative d'un seuil de détection des tachycardies.

10. Le dispositif de la revendication 1, dans lequel la délivrance d'un indicateur de suspicion de fracture (20) est conditionnée à la détection (16) d'une séquence de dépolarisations présentant des intervalles de couplage successifs courts et variables.

11. Le dispositif de la revendication 1, dans lequel la délivrance d'un indicateur de suspicion de fracture (20) est conditionnée à la détection (18) d'une séquence de dépolarisations présentant une amplitude de signal inférieure à un seuil de détection prédéterminé.

12. Le dispositif de la revendication 1, dans lequel le capteur d'accélération est un capteur apte à relever l'accélération au niveau d'un ventricule du coeur.

13. Le dispositif de la revendication 1, dans lequel le capteur d'accélération est un capteur apte à relever l'accélération au niveau d'une oreillette du coeur.

14. Le dispositif de la revendication 1, dans lequel le capteur d'accélération est un capteur apte à relever l'accélération au niveau d'un vaisseau sanguin périphérique du coeur.

## Claims

1. An active implantable medical device of the cardiac prosthesis type for pacing, resynchronization, cardioversion and/or defibrillation, comprising:
- rhythm sensing means, comprising an implantable lead provided with at least one endocardial electrode adapted to collect electrical potentials representative of myocardium depolarisations, and a sensing circuit adapted to analyse the collected potentials and issue a sequence of signals that are representative of the successive ventricular and atrial depolarisations (P, R); and
- means for detecting myocardium contractions, comprising an endocardial acceleration sensor, and means for determining at least one peak of endocardial acceleration over one given cardiac cycle and issue a sequence of signals that are representative of the successive endocardial acceleration peaks (PEA I),
said device being **characterised by** further comprising means for detecting a fracture of said lead, comprising means for:
- receiving as input, and correlating together, said signals representative of the cardiac depolarisations and said signals representative of the acceleration peaks, and
- in case of non-correlation, issuing an indicator of suspicion of a fracture (20) of said lead.

2. The device of claim 1, comprising two distinct endocardial leads, a first lead being equipped with said endocardial electrode and a second lead being equipped with said endocardial acceleration sensor, said means of fracture detection being means for detecting a fracture of said first lead.

3. The device of claim 1, further comprising means for measuring lead impedance (22) in response to the issuance of an indicator of lead fracture suspicion.

4. The device of claim 1, further comprising means for generating an alarm signal (26) in response to a recurrent issuance of indicators of lead fracture suspicion over a predetermined duration.

5. The device of claim 4, wherein the means for generating an alarm signal (26) comprise means for recording markers in a memory of the device, said memory being readable by an external programmer.

6. The device of claim 4, wherein the means for generating an alarm signal (26) comprise RF transmission means.

7. The device of claim 4, wherein the means for generating an alarm signal (26) comprise means for generating an audible signal.

8. The device of claim 1, wherein the issuance of an indicator of lead fracture suspicion (20) is conditioned on the detection (12) of a sequence of acceleration peaks that is stable in terms of amplitude and/or coupling intervals.

9. The device of claim 1, wherein the issuance of an indicator of lead fracture suspicion (20) is conditioned on the detection (14) of a sequence of acceleration peaks with a frequency that is lower than a limit frequency representative of a threshold for the detection of tachycardia.

10. The device of claim 1, wherein the issuance of an indicator of lead fracture suspicion (20) is conditioned on the detection (16) of a sequence of depolarisations showing successive short and variable coupling intervals.

11. The device of claim 1, wherein the issuance of an indicator of lead fracture suspicion (20) is conditioned on the detection (18) of a sequence of depolarisations showing a signal amplitude lower than a predetermined sensing threshold.

12. The device of claim 1, wherein the acceleration sensor is a sensor adapted to measure the acceleration at a ventricle of the heart.

13. The device of claim 1, wherein the acceleration sensor is a sensor adapted to measure the acceleration at an atrium of the heart.

14. The device of claim 1, wherein the acceleration sensor is a sensor adapted to measure acceleration level at a blood vessel that is peripheral to the heart.

## Patentansprüche

1. Aktive medizinische implantierbare Vorrichtung vorn Typ Prothese zur Stimulation, Resynchronisation, Kardioversion und/oder Defibrillation des Herzens, aufweisend:
- Mittel zur Rhythmuserfassung, die eine implantierbare Sonde, die mit wenigstens einer endokavitären Elektrode ausgestattet ist, die dazu geeignet ist, elektrische Potentiale, die für die Depolarisationen des Herzmuskels repräsentativ sind, aufzunehmen und einen Erfassungsschaltkreis, der geeignet ist, die aufgenommenen Potentiale zu analysieren und eine Folge an Signalen, die für aufeinanderfolgende ventrikuläre und aurikuläre Depolarisationen (P, R) repräsentativ sind, auszugeben, aufweisen, und
- Mittel zur Erfassung der Herzmuskelkontraktionen, die einen Sensor der endokardialen Beschleunigung und Mittel zum Bestimmen wenigstens einer endokardialen Beschleunigungsspitze während eines gegebenen Herzzyklus und Ausgeben einer Folge an Signalen, die für aufeinanderfolgende Beschleunigungsspitzen (PEA 1) repräsenativ sind, aufweisen
wobei die Vorrichtung **dadurch** charakterisiert ist, dass sie darüber hinaus Mittel zur Erfassung der Fraktur der genannten Sonde aufweisen, die Mittel aufweisen zum:
- Empfangen am Eingang und untereinander korrelieren der genannten für Depolarisationen repräsentativen Signale und der genannten für Beschleunigungsspitzen repräsentativen Signale und
- falls keine Korrelation vorliegt, Ausgeben eines Indikators des Verdachts auf Fraktur (20) der genannten Sonde.

2. Vorrichtung nach Anspruch 1, die zwei verschiedene endokavitäre Sonden mit einer ersten Sonde, die die genannte endokavitäre Elektrode trägt, und mit einer zweiten Sonde, die den genannten Sensor der endokardialen Beschleunigung trägt, aufweist und wobei die genannten Mittel zur Erfassung der Fraktur Mittel zur Erfassung einer Fraktur der genannten ersten Sonde sind.

3. Vorrichtung nach Anspruch 1, die darüber hinaus Mittel zur Messung der Impedanz der Sonde (22) aufweist, die in Antwort auf die Ausgabe eines Indikators des Verdachts auf Fraktur aktivierbar sind.

4. Vorrichtung nach Anspruch 1, die darüber hinaus Mittel zum Erzeugen eines Alarmsignals (26) in Antwort auf die wiederkehrende Ausgabe der Indikatoren des Verdachts auf Fraktur über eine vorbestimmte Dauer hinweg, aufweisen.

5. Vorrichtung nach Anspruch 4, in welcher die Mittel zum Erzeugen eines Alarmsignals (26) Mittel zur Aufzeichnung von Markern in einem Speicher der Vorrichtung, die für einen externen Programmgestalter lesbar sind, aufweisen.

6. Vorrichtung nach Anspruch 4, in welcher die Mittel um Erzeugen eines Alarmsignals (26) Mittel zur RF-Übertragung aufweisen.

7. Vorrichtung nach Anspruch 4, in welcher die Mittel zum Erzeugen eines Alarmsignals (26) Mittel zur Erzeugung eines sonoren Signals aufweisen.

8. Vorrichtung nach Anspruch 1, in welcher die Ausgabe eines Indikators des Verdachts auf Fraktur (20) auf die Erfassung (12) einer Folge an Beschleunigungsspitzen, die in Amplitude und/oder in Intervallen der Kopplung stabil sind, hin eingerichtet ist ist.

9. Vorrichtung nach Anspruch 1, in welcher die Ausgabe eines Indikators des Verdachts auf Fraktur (20) auf die Erfassung (14) einer Folge an Beschleunigungsspitzen mit geringerer Frequenz als eine Grenzfrequenz, die für einen Grenzwert der Erfassung von Tachykardien repräsentativ ist, hin eingerichtet ist.

10. Vorrichtung nach Anspruch 1, in welcher die Ausgabe eines Indikators des Verdachts auf Fraktur (20) auf die Erfassung (16) einer Folge an Depolarisationen, die kurze und variable aufeinanderfolgende Intervalle der Kopplung aufweisen, hin eingerichtet ist.

11. Vorrichtung nach Anspruch 1, in welcher die Ausgabe eines Indikators des Verdachts auf Fraktur (20) auf die Erfassung (18) einer Folge an Depolarisationen, die eine geringere Signalamplitude als ein vorbestimmter Erfassungsgrenzwert aufweisen, hin eingerichtet ist.

12. Vorrichtung nach Anspruch 1, in welcher der Sensor der Beschleunigung ein Sensor ist, der dazu geeignet ist, die Beschleunigung auf dem Niveau eines Herzventrikels zu erfassen.

13. Vorrichtung nach Anspruch 1, in welcher der Sensor der Beschleunigung ein Sensor ist, der dazu geeignet ist, die Beschleunigung auf dem Niveau eines Herzvorhofes zu erfassen.

14. Vorrichtung nach Anspruch 1, in welcher der Sensor der Beschleunigung ein Sensor ist, der dazu geeignet ist, die Beschleunigung auf dem Niveau eines Herzrandgefäßes zu erfassen.
